(19) Europäisches Patentamt
European Patent Office
Office européen des brevets

(11) Numéro de publication : **0 433 149 B1**

(12) # FASCICULE DE BREVET EUROPEEN

(45) Date de publication du fascicule du brevet : **16.02.94 Bulletin 94/07**

(21) Numéro de dépôt : **90403502.9**

(22) Date de dépôt : **10.12.90**

(51) Int. Cl.$^5$ : **C07D 513/06,** C07D 209/92, C07D 417/06, C07D 211/70, C07D 295/13, C07D 471/06, A61K 31/445, A61K 31/495, A61K 31/54, // (C07D513/06, 285:00, 221:00), (C07D471/06, 235:00, 221:00)

(54) **Antagonistes de la sérotonine, leur préparation et les médicaments les contenant.**

(30) Priorité : **13.12.89 FR 8916459**
**05.06.90 FR 9006943**

(43) Date de publication de la demande : **19.06.91 Bulletin 91/25**

(45) Mention de la délivrance du brevet : **16.02.94 Bulletin 94/07**

(84) Etats contractants désignés : **AT BE CH DE DK ES FR GB GR IT LI LU NL SE**

(56) Documents cités :
**EP-A- 0 022 118**
**EP-A- 0 330 065**
**EP-A- 0 332 528**
**EP-A- 0 350 403**
**US-A- 3 303 189**
**US-A- 4 110 449**

(73) Titulaire : **RHONE-POULENC SANTE**
**20, avenue Raymond Aron**
**F-92160 Antony (FR)**

(72) Inventeur : **Comte, Marie-Thérèse**
**1 allée Costes et Bellonte**
**F-94550 Chevilly Larue (FR)**
Inventeur : **Gueremy, Claude**
**3 rue Daumesnil**
**F-78800 Houilles (FR)**
Inventeur : **Malleron, Jean-Luc**
**2 allée Renoir**
**F-91460 Marcoussis (FR)**
Inventeur : **Peyronnel, Jean-François**
**36 Parc d'Ardenay**
**F-91120 Palaiseau (FR)**
Inventeur : **Truchon, Alain**
**73 rue Henri Gorjus**
**F-69004 Lyon (FR)**

(74) Mandataire : **Savina, Jacques et al**
**RHONE-POULENC RORER S.A., Direction des Brevets, 90 avenue Raymond Aron**
**F-92165 Antony Cédex (FR)**

**EP 0 433 149 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés de formule :

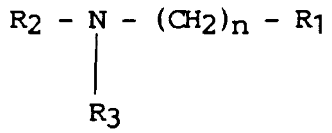

(I)

leurs sels, leurs procédés de préparation et les médicaments les contenant.

Dans la formule (I) :

- $R_1$ représente
  . un radical tétrahydro-1, 2, 3, 6 pyridyl-1 substitué en position -4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
  . un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1, 2 yl-3, (d) un radical benzisoxazol-1,2 yl-3 ou (e) un radical pyridyl-2,
  . un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl-1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3, (j) un radical indolyl-3 substitué sur l'atome d'azote par un radical alkyle ou alkylcarbonyle et/ou en position -5 par un atome de chlore ou de fluor ou (k) un radical (hydroxy-5 indolyl)-3,
- $R_2$ représente un radical $SO_2R_4$ dans lequel $R_4$ représente un radical alkyle ou phényle,
- $R_3$ représente un radical phényle ou naphtyle,
- ou bien $R_2$ et $R_3$ forment ensemble avec l'atome d'azote auquel ils sont attachés un cycle choisi parmi les formules:

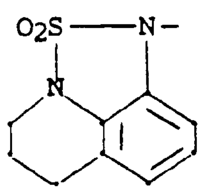

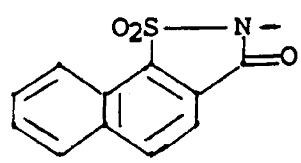

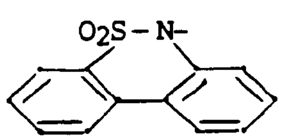

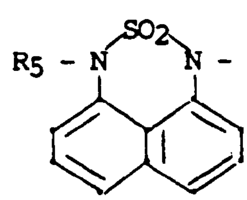

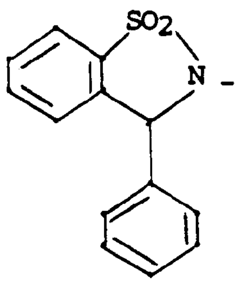 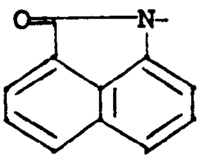 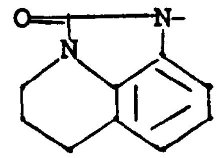

- $R_5$ représente un radical alkyle ou une chaîne $-(CH_2)_n-R_1$,
- n est égal à 2, 3 ou 4.

Dans les définitions qui précèdent et celles qui seront citées ci-après, les radicaux alkyle contiennent 1 à 4 atomes de carbone en chaîne droite ou ramifiée et les atomes d'halogène sont de préférence les atomes de fluor, de chlore ou de brome.

L'invention concerne également les sels des composés de formule (I) avec les acides minéraux ou organiques.

Les composés de formule (I) à l'exception de ceux pour lesquels $R_1$ représente un radical aminophényl-4 pipérazinyl-1, peuvent être préparés par action d'un dérivé de formule:

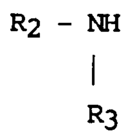 (II)

dans laquelle $R_2$ et $R_3$ ont les mêmes significations que dans la formule (I), sur un dérivé halogéné de formule:

$$Hal - (CH_2)_n - R_1 \qquad (III)$$

dans laquelle Hal représente un atome d'halogène, n a les mêmes significations que dans la formule (I) et $R_1$ a les mêmes significations que ci-dessus.

Cette réaction s'effectue, de préférence, en présence d'une base telle qu'un hydrure de métal alcalin, un hydroxyde de métal alcalin ou un carbonate de métal alcalin, au sein d'un solvant inerte tel que le diméthylformamide ou le tétrahydrofuranne, à une température comprise entre 20°C et la température d'ébullition du solvant.

Les composés de formule (II) peuvent être préparés par application ou adaptation des méthodes décrites par H. P. KAUFMANN et coll., Ber., 6, 1499 (1922); F. ULLMANN et coll., Chem. Ber. 43, 2684 (1910) et C.W. REES et coll., J. Chem. Soc., 993 (1971) et des méthodes décrites dans les exemples.

Les dérivés halogénés de formule (III) peuvent être obtenus par action d'une amine de formule:

$$HR_1 \qquad (IV)$$

dans laquelle $R_1$ a les mêmes significations que dans la formule (III) sur un dérivé dihalogéné de formule:

$$Hal - (CH_2)_n - X \qquad (V)$$

dans laquelle Hal et X représentent un atome d'halogène et n est égal à 2, 3 ou 4.

Cette réaction s'effectue généralement au sein d'un solvant inerte tel que le diméthylformamide ou l'acétonitrile, en présence d'une base tel qu'un carbonate de métal alcalin, à une température comprise entre 20°C la température d'ébullition du solvant.

Les amines de formule (IV) sont commercialisées ou peuvent être obtenues par application ou adaptation des méthodes décrites par R. L. DUNCAN et coll., J. Med. Chem., 13, 1 (1970); L. NEDELEC et coll., Eur. J. Med. Chem. 22, 33 (1987); D.K. YUNK et coll., J. Med. Chem., 21, 1301 (1978); J.P. YEVICH et coll., J. Med. Chem., 29, 3, 359 (1986); L. THUNUS et coll., Ann. Pharm., 38, 353 (1980); L. GOOTES et coll., Arzneim Forsch, 17, 1145 (1967); J. BERGMAN et coll., J. Het. Chem., 1071 (1970) et dans les brevets DE 2139084, BE 62 630, EP 110 435, US 4 470 989 et US 3 575 990 et des méthodes décrites dans les exemples.

Les composés de formule (I) à l'exception de ceux pour lesquels $R_1$ représente un radical aminophényl-4 pipérazinyl-1, peuvent également être obtenus par action d'un dérivé de formule :

$$R_2 - \underset{\substack{| \\ R_3}}{N} - (CH_2)_n - Hal \qquad (VI)$$

dans laquelle $R_2$, $R_3$ et n ont les mêmes significations que dans la formule (I) et Hal représente un atome d'halogène sur un dérivé de formule (IV) dans laquelle $R_1$ a les mêmes significations que ci-dessus.

Cette réaction s'effectue généralement au sein d'un solvant inerte tel que le tétrahydrofuranne ou le diméthylformamide, en présence d'une base telle qu'un bicarbonate de métal alcalin ou une triéthylamine, à la température d'ébullition du milieu réactionnel.

Les dérivés de formule (VI) peuvent être obtenus par action d'un dérivé de formule (II) sur un dérivé dihalogéné de formule (V).

Cette réaction s'effectue dans un solvant inerte tel que le diméthylformamide, au moyen d'hydrure de sodium à une température comprise entre 20°C et la température d'ébullition du solvant.

Les composés de formule (I) pour lesquels $R_1$ représente un radical aminophényl-4 pipérazinyl-1 peuvent être obtenus par réduction des composés de formule (I) correspondants pour lequels $R_1$ représente un radical nitrophényl-4 pipérazinyl-1.

Cette réduction s'effectue généralement au moyen de chlorure stanneux et de borohydrure de sodium, dans un alcool tel que le méthanol ou l'éthanol, à une température comprise entre 20 et 70°C, ou au moyen de fer et d'acide chlorhydrique dans l'eau ou un mélange eau-alcool, à une température comprise entre 20°C et la température de reflux du mélange réactionnel.

Les mélanges réactionnels obtenus par les divers procédés décrits précédemment sont traités suivant des méthodes classiques physiques (évaporation, extraction, distillation, cristallisation, chromatographie...) ou chimiques (formation de sels,...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés intéressantes. Ces composés possèdent des propriétés antagonistes de la sérotonine (récepteurs 5 HT2) et sont donc utiles pour le traitement des affections où la sérotonine est impliquée, notamment les affections du système nerveux central, du système cardiovasculaire et les troubles gastrointestinaux.

Ces composés sont, en particulier, utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'aggrégation plaquettaire.

L'affinité des composés de formule (I) pour les sites récepteurs centraux à sérotonine (type S2) a été déterminée selon une technique inspirée de celle de J. E. LEYSEN et coll., Mol. Pharmacol., 21, 301 (1982) qui consiste à mesurer l'affinité des produits pour les sites de liaison de la kétansérine tritiée. Dans ce test, la $CI_{50}$ des composés de formule (I) est généralement inférieure à 25 nM.

Les composés de formule (I) présentent une toxicité faible. Ils sont généralement atoxiques à 300 mg/kg par voie orale chez la souris en administration unique.

Sont particulièrement intéressants les composés pour lesquels $R_1$ représente un radical phényl-4 tétrahydro-1,2,3,6 pyridyl-1, phényl-4 pipéridino, (fluoro-4 benzoyl)-4 pipéridino ou phényl-4 pipérazinyl-1 dont le noyau phényle est éventuellement substitué par un atome d'halogène ou un radical hydroxy.

Sont également intéressants les composés pour lesquels n est égal à 3.

Sont également intéressants les composés pour lesquels $R_2$ et $R_3$ forment avec l'atome d'azote auquel ils sont rattachés un cycle dihydro-5,6 1H, 4H-1,2,5-thiadiazolo [4,3,2-ij] quinoléine dioxyde-2,2 ou phényl-3 benzisothiazole-1,2 dioxyde-1,1.

D'un intérêt particulier sont les composés suivants :

- [(phényl-4 tétrahydro-1,2,3,6 pyridyl-1)-3 propyl]-1 dihydro-5,6 1H, 4H-1,2,5-thiadiazolo [4,3,2-ij] quinoléine dioxyde-2,2
- [(fluoro-4 phényl)-4 pipérazinyl)-3 propyl]-1 dihydro-5,6 1H, 4H-1,2,5-thiadiazolo [4,3,2-ij] quinoléine dioxyde-2,2
- [(hydroxy-4 phényl)-4 pipérazinyl)-3 propyl]-1 dihydro-5,6 1H, 4H-1,2,5-thiadiazolo [4,3,2-ij] quinoléine dioxyde-2,2
- [(fluoro-4 phényl)-4 pipérazinyl-1)-3 propyl]-2 phényl-3-benzisothiazole-1,2 dioxyde-1,1-(3RS).

Pour l'emploi thérapeutique, il peut être fait usage des composés de formule (I) tels quels ou à l'état de sels pharmaceutiquement acceptables.

Comme sels pharmaceutiquement acceptables, peuvent être notamment cités les sels d'addition avec les acides minéraux tels que chlorhydrates, sulfates, nitrates, phosphates ou organiques tels que acétates, propionates, succinates, oxalates, benzoates, fumarates, maléates, méthanesulfonates, iséthionates, théophilline- acétates, salicylates, phénolphtalinates, méthylène-bis-β-oxynaphtoates ou des dérivés de substitution de ces dérivés.

Les exemples suivants donnés à titre non limitatif montrent comment l'invention peut être mise en pratique.

EXEMPLE 1

Une solution de 2,6 g de dihydro-5,6 1H, 4H-1,2,5-thiadiazolo [4,3,2-ij]quinoléine dioxyde-2,2 dans 10 $cm^3$ de N,N-diméthyl formamide sec est ajoutée goutte à goutte à une suspension de 0,45 g d'hydrure de sodium (suspension à 80% dans l'huile) dans 20 $cm^3$ de N,N-diméthyl formamide. Après 15 minutes d'agitation, on ajoute une solution de 3,51 g de (chloro-3 propyl)-1 phényl-4 tétrahydro-1,2,3,6 pyridine dans 10 $cm^3$ de N,N-diméthyl formamide. Le mélange réactionnel est chauffé 90 minutes à 80°C, puis refroidi et versé dans un mélange de 100 $cm^3$ d'eau et 200 $cm^3$ d'acétate d'éthyle. La phase organique est lavée à l'eau, séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 50 cm) en éluant par de l'acétate d'éthyle et en recueillant des fractions de 125 $cm^3$. Les fractions 2 à 6 sont réunies et concentrées à sec sous pression réduite (2,7 kPa). Après recristallisation dans l'acétate d'éthyle on obtient 3,7 g de [(phényl-4 tétrahydro-1,2,3,6 pyridyl-1)-3 propyl]-1 dihydro-5,6 1H, 4H-1,2,5-thiadiazolo [4,3,2-ij] quinoléine dioxyde-2,2 fondant à 120°C.

Le dihydro-5,6 1H, 4H-1,2,5-thiadiazolo[4,3,2-ij] quinoléine dioxyde-2,2 peut être préparé de la manière suivante: à une solution de 9 g d'amino-8 tétrahydro-1,2,5,6 quinoléine 90 $cm^3$ de diglyme on ajoute 6 g de sulfamide et l'on chauffe à 160°C pendant 90 minutes. Le mélange réactionnel est refroidi puis dilué dans un mélange de 300 $cm^3$ d'eau et 300 $cm^3$ d'acétate d'éthyle. La phase organique est lavée à l'eau (3 fois 300 $cm^3$), séchée sur sulfate de magnésium et concentrée à sec sous pression réduite (2,7 kPa). Le résidu est chromatographié sur une colonne de gel de silice (granulométrie 0,2-0,063 mm, diamètre 6 cm, hauteur 60 cm) en éluant sous une pression de 0,7 bar d'azote par un mélange de cyclohexane et d'acétate d'éthyle (80-20 en volumes) et en recueillant des fractions de 125 $cm^3$. Les fractions 12 à 22 sont réunies et concentrées à sec sous pression réduite (2,7 kPa) pour donner 9,4g de dihydro-5,6 1H, 4H-1,2,5-thiadiazolo[4,3,2-ij] quinoléine dioxyde-2,2 fondant à 96°C.

L'amino-8 tétrahydro-1,2,5,6 quinoléine peut être préparée selon la méthode décrite par HAZLEWOOD et coll. J. Pr. Soc., N.S. WALES, 71, 462 (1937-1938).

La (chloro-3 propyl)-1 phényl-4 tétrahydro-1,2,4,6 pyridine peut être préparée de la manière suivante: une solution de 6 $cm^3$ de bromo-1 chloro-3 propane et 5,1 g de phényl-4 tétrahydro-1,2,3,6 pyridine dans 60 $cm^3$ d'acétonitrile est agitée 20 heures à 25 °C avec 97 g de carbonate de potassium. Le mélange est filtré puis concentré à sec sous pression réduite (2,7 kPa). Le résidu est chromatographié sur une colonne de gel de silice (granulométrie 0,2-0,063 mm, diamètre 4 cm, hauteur 40 cm) en éluant par un mélane de cyclohexane et d'acétate d'éthyle (50-50 en volume) et en recueillant des fractions de 250 $cm^3$. Les fractions 7 à 13 sont réunies et concentrées à sec sous pression réduite (2,7 kPa) pour donner 5 g de (chloro-3 propyl)-1 phényl-4 tétrahydro-1,2,3,6 pyridine sous la forme d'une huile jaune.

R.M.N. du proton (CDCl3) :

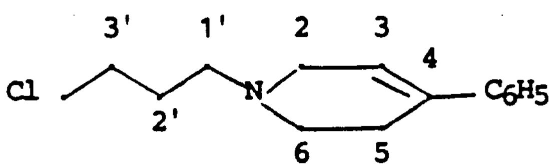

| | | | |
|---|---|---|---|
| δ7,35 | bd | 2H | $C_6H_5$ ortho |
| δ7,28 | bt | 2H | $C_6H_5$ méta |
| δ7,18 | bt | 1H | $C_6H_5$ para |
| δ6 | bs | 1H | CH éthylique ($H_3$) |
| δ3,6 | t | 2H | $CH_2Cl$ ($2xH_3$') |
| δ3,1 | bs | 2H | $CH_2$-N ($2xH_2$') |
| δ2,7 | t | 2H | $CH_2$-N ($2xH_1$') |
| δ2,55 | m | 4H | $CH_2$ ($2xH_6+2H_5$) |
| δ2 | m | 2H | $CH_2$ ($2xH_2$') |

EXEMPLE 2

Une solution de 5,34 g de dihydro-5,6 1H, 4H-1,2,5-thiadiazolo [4,3,2-ij]quinoléine dioxyde-2,2 dans 50 $cm^3$ de N,N-diméthyl formamide sec est ajoutée goutte à goutte à une suspension de 0,72 g d'hydrure de sodium (suspension à 80% dans l'huile) dans 20 $cm^3$ de N,N-diméthyl formamide. Après 15 minutes d'agitation, on ajoute 7,68 g de (chloro-3 propyl)-1 (fluoro-4 phényl)-4 pipérazine. Le mélange réactionnel est chauffé 1 heure et 30 minutes à 100°C, puis refroidi et versé dans un mélange de 300 $cm^3$ d'eau et 500 $cm^3$ d'acétate d'éthyle. La phase organique est lavée à l'eau (3 fois 200 $cm^3$), séchée sur sulfate de magnésium et concentrée à sec sous pression réduite (2,7 kPA). Le résidu est chromatographié sur une colonne de gel de silice (granulométrie 0,2-0,063 mm, diamètre 6 cm, hauteur 50 cm) en éluant sous une pression de 0,7 bar d'azote par un mélange de cyclohexane et d'acétate d'éthyle (80-20 en volumes) et en recueillant des fractions de 125 $cm^3$. Les fraction 10 à 28 sont réunies et concentrées à sec sous pression réduite (2,7 kPA). Le solide obtenu est recristallisé dans 80 $cm^3$ d'éthanol. On obtient 7,2 g de [((fluoro-4 phényl)-4 pipérazinyl)-3 propyl]-1 dihydro-5,6 1H, 4H-1,2,5-thiadiazolo [4,3,2-ij] quinoléine dioxyde-2,2 fondant à 98°C.

La (chloro-3 propyl)-1 (fluoro-4 phényl)-4 pipérazine peut être obtenue de la manière suivante: une solution de 68 $cm^3$ de bromo-1 chloro-3 propane et 50 g de (fluoro-4 phényl)-4 pipérazine dans 400 $cm^3$ d'acétonitrile est agitée 20 heures à 25°C avec 97 g de carbonate de potassium. Le mélange est filtré puis concentré à sec sous pression réduite (2,7 kPa). Le résidu est chromatographié sur une colonne de gel de silice (granulométrie 0,2-0,063 mm, diamètre 9 cm, hauteur 60 cm) en éluant par de l'acétate d'éthyle et en recueillant des fractions de 500 $cm^3$. Les fractions 5 à 7 sont sont réunies et concentrées à sec sous pression réduite (2,7 kPa) pour donner 44,4 g de (chloro-3 propyl)-1 (fluoro-4 phényl)-4 pipérazine sous la forme d'une huile jaune.

R.M.N. du proton (CDCl3) :

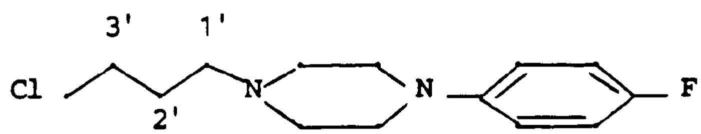

| | | | |
|---|---|---|---|
| δ6,8 à 6,95 | m | 4H | aromatiques |
| δ3,6 | t | 2H | $CH_2Cl$ ($2xH_3$') |
| δ3,1 | m | 4H | $2CH_2$-N |
| δ2,6 | m | 4H | $2CH_2$-N |
| δ2,5 | t | 2H | $CH_2N$ ($2xH_1$') |
| δ2 | m | 2H | $CH_2$ ($2xH_2$') |

EXEMPLE 3

Une solution de 4 g de (chloro-3 propyl)-1 dihydro-5,6 1H, 4H-1,2,5-thiadiazolo [4,3,2-ij]quinoléine dioxyde-2,2, 4,75 g d'(hydroxy-4 phényl)-4 pipérazine et 5,88 $cm^3$ de triéthylamine dans 50 $cm^3$ de N,N-diméthyl

formamide sec est chauffée 90 minutes au reflux puis refroidie et versée dans un mélange de 300 $cm^3$ d'eau et 300 $cm^3$ d'acétate d'éthyle. La phase organique est lavée à l'eau (3 fois 200 $cm^3$), séchée sur sulfate de magnésium et concentrée à sec sous pression réduite (2,7 kPa). Le solide est recristallisé dans 300 $cm^3$ d'acétate d'éthyle et séché. On obtient 2,8 g d'[((hydroxy-4 phényl)-4 pipérazinyl-1)-3 propyl]-1 dihydro-5,6 1H, 4H-1,2,5-thiadiazolo[4,3,2-ij]quinoléine dioxyde-2,2 fondant à 182°C.

Le (chloro-3 propyl)-1 dihydro-5,6 1H, 4H-1,2,5-thiadiazolo [4,3,2-ij] quinoléine dioxyde-2,2 peut être obtenu de la manière suivante: une solution de 4 g de dihydro-5,6 1H, 4H-1,2,5-thiadiazolo [4,3,2-ij]quinoléine dioxyde-2,2 dans 30 $cm^3$ de N,N-diméthyl formamide sec est ajoutée goutte à goutte à une suspension de 0,69 g d'hydrure de sodium (suspension à 80% dans l'huile) dans 20 $cm^3$ de N,N-diméthyl formamide. Après 30 minutes d'agitation, on ajoute une solution de 3, 25 g de bromo-1 chloro-3 propane dans 20 $cm^3$ de N,N-diméthyl formamide. Le mélange réactionnel est agité 2 heures à 25°C puis dilué dans un mélange de 300 $cm^3$ d'eau et 300 $cm^3$ d'acétate d'éthyle. La phase organique est lavée à l'eau (3 fois 200 $cm^3$), séchée sur sulfate de magnésium et concentrée à sec sous pression réduite (2,7 kPA). Le résidu est chromatographié sur une colonne de gel de silice (granulométrie 0,2-0,063 mm, diamètre 4 cm, hauteur 50 cm) en éluant sous une pression de 0,7 bar d'azote par un mélange de cyclohexane et d'acétate d'éthyle (80-20 en volumes) et en recueillant des fractions de 125 $cm^3$. Les fractions 4 à 12 sont réunies et concentrées à sec sous pression réduite (2,7 kPA) pour donner 4,2 g de (chloro-3 propyl)-1 dihydro-5,6 1H, 4H-1,2,5-thiadiazolo [4,3,2-ij]quinoléine dioxyde-2,2 sous la forme d'une huile jaune.

R.M.N. du proton (CDCl3) :

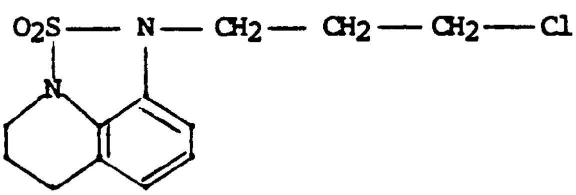

| | | | |
|---|---|---|---|
| δ6,95 | t | 1H | ) 3 aromatiques |
| δ6,8 | bd | 1H | ) |
| δ6,73 | bd | 1H | ) |
| δ3,95 | t | 2H | $CH_2$ - N - $SO_2$ |
| δ3,75 | m | 4H | $CH_2Cl$ et $CH_2$-N-$SO_2$ |
| δ2,80 | t | 2H | $CH_2$-< |
| δ2,35 et 2,20 | | | 2xm, 2x$CH_2$ ($CH_2$ centraux) |

Le dibromhydrate d'(hydroxy-4 phényl)-1 pipérazine peut être préparé de la manière suivante: à 70 g de dichlorhydrate de (méthoxy-4 phényl)-4 pipérazine sont ajoutés, durant 30 minutes et à une température voisine de 20°C, 720 $cm^3$ d'une solution aqueuse d'acide bromhydrique à 47%. Le mélange est chauffé à ébullition pendant 4 heures puis refroidi à une température voisine de 20°C. Le mélange est concentré à 40°C sous pression réduite (20 mm de mercure; 2,7 kPA). L'huile résiduelle est reprise par 300 $cm^3$ d'acétonitrile. Le précipité est séparé par filtration, lavé par 2 fois 50 $cm^3$ d'acétonitrile et 2 fois 100 $cm^3$ d'éther de diisopropyle. On obtient 85,2 g de dibromhydrate d'(hydroxy-4 phényl)-4 pipérazine (point de fusion supérieur à 260°C) utilisé à l'état brut dans les synthèses ultérieures.

EXEMPLE 4

Une solution de 3,4 g de (chloro-2 éthyl)-1 dihydro-5,6 1H, 4H-1,2,5-thiadiazolo [4,3,2-ij]quinoléine dioxyde-2,2, 2,25 g de (fluoro-4 phényl)-4 pipérazine et 1,75 $cm^3$ de triéthylamine dans 50 $cm^3$ de N,N-diméthyl formamide sec est chauffée 3 heures au reflux puis refroidie et versée dans un mélange de 300 $cm^3$ d'eau et 300 $cm^3$ d'acétate d'éthyle. La phase organique est lavée à l'eau (3 fois 200 $cm^3$), séchée sur sulfate de magnésium et concentrée à sec sous pression réduite (2,7 kPA). Le résidu est chromatographié sur une colonne de gel de silice (granulométrie 0,2-0,063 mm, diamètre 4 cm, hauteur 50 cm) en éluant sous une pression de 0,7 bar d'azote par un mélange de cyclohexane et d'acétate d'éthyle (80-20 en volumes) et en recueillant des fractions de 125 $cm^3$. Les fractions 33 à 43 sont réunies et concentrées à sec sous pression réduite (2,7 kPa). Après recristallisation dans 50 $cm^3$ d'oxyde d'isopropyle on obtient 0,85 g de [((fluoro-4 phényl)-4 pipérazinyl-1)-2 éthyl]-1 dihydro-5,6 1H, 4H-1,2,5-thiadiazolo[4,3,2-ij]quinoléine dioxyde-2,2 fondant à 118°C.

Le (chloro-2 éthyl)-1 dihydro-5,6 1H,4H-1,2,5-thiadiazolo [4,3,2-ij] quinoléine dioxyde-2,2 peut être préparé de la manière suivante: une solution de 4 g de dihydro-5,6 1H, 4H-1,2,5-thiadiazolo [4,3,2-ij]quinoléine dioxyde-2,2 dans 20 $cm^3$ de N,N-diméthyl formamide sec est ajoutée goutte à goutte à une suspension de 0,69 g d'hydrure de sodium (suspension à 80% dans l'huile) dans 20 $cm^3$ de N,N-diméthyl formamide. Après 15 minutes d'agitation, on ajoute une solution de 3,29 g de bromo-1 chloro-2 éthane dans 10 $cm^3$ de N,N-diméthyl formamide. Le mélange réactionnel est agité 3 heures à 20°C puis dilué dans un mélange de 300 $cm^3$ d'eau et 300 $cm^3$ d'acétate d'éthyle. La phase organique est lavée à l'eau (3 fois 200 $cm^3$), séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 40 cm) en éluant sous une pression de 0,7 bar d'azote par un mélange de cyclohexane et d'acétate d'éthyle (80-20 en volumes) et en recueillant des fractions de 125 $cm^3$. Les fractions 8 à 16 sont réunies et concentrées à sec sous pression réduite (2,7 kPa) pour donner 3,4 g de (chloro-2 éthyl)-1 dihydro-5,6 1H, 4H-1,2,5-thiadiazolo [4,3,2-ij]quinoléine dioxyde-2,2 fondant à 70°C.

## EXEMPLE 5

On opère comme à l'exemple 3, à partir de 3,8 g de (chloro-3 propyl)-1 dihydro-5,6 1H, 4H-1,2,5-thiadiazolo [4,3,2-i,j] quinoléine dioxyde-2,2, de 2,9 g de (fluoro-5 indolyl-3)-4 tétrahydro-1,2,3,6 pyridine et de 3,3 g de bicarbonate de sodium dans un mélange de 40 $cm^3$ de diméthylformamide et de 25 $cm^3$ de tétrahydrofuranne. Le mélange est chauffé à ébullition durant 48 heures puis refroidi à une température voisine de 20°C. Après purification par flash-chromatographie sur colonne de silice, sous courant d'argon à moyenne pression (0,5-1,5 bar) avec de l'acétate d'éthyle comme éluant et recristallisation dans 200 $cm^3$ d'acétonitrile bouillant, on obtient 2,5 g de [((fluoro-5 indolyl-3)-4 tétrahydro-1,2,3,6 pyridyl-1)-3 propyl]-1 dihydro-5,6 1H, 4H-1,2,5-thiadiazolo[4,3,2-i,j] quinoléine dioxyde-2,2 fondant à 178 °C.

La (fluoro-5 indolyl-3)-4 tétrahydro-1,2,3,6 pyridine peut être préparée selon la méthode décrite par L. NEDELEC et coll., Eur. J. Med. Chem., 22, 33 (1987).

## EXEMPLE 6

Une solution de 4,5 g de (chloro-4 butyl)-1 dihydro-5,6 1H, 4H-1,2,5-thiadiazolo [4,3,2-ij] quinoléine dioxyde-2,2, 2,7 g de (fluoro-4 phényl)-4 pipérazine et 2,1 $cm^3$ de triéthylamine dans 45 $cm^3$ de N-N dyméthyl formamide sec est chauffé 90 minutes au reflux puis refroidie et versée dans un mélange de 300 $cm^3$ d'eau et 300 $cm^3$ d'acétate d'éthyle. La phase organique est lavée à l'eau (3 fois 200 $cm^3$), séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 50 cm) en éluant, sous une pression de 0,7 bar d'azote, par un mélange de cyclohexane et d'acétate d'éthyle (30-70 en volumes) et en recueillant des fractions de 125 $cm^3$. Les fractions 7 à 16 sont réunies et concentrées à sec sous pression réduite (2,7 kPa). Après recristallisation dans 40 $cm^3$ d'oxyde d'isopropyle on obtient 2,7 g de [((fluoro-4 phényl)-4 pipérazinyl-1)-4 butyl]-1 dihydro-5,6 1H, 4H-1,2,5-thiadiazolo [4,3,2-ij] quinoléine dioxyde-2,2 fondant à 87°C.

Le (chloro-4 butyl)-1 dihydro-5,6 1H, 4H-1,2,5-thiadiazolo [4,3,2-ij] quinoléine peut être préparé de la manière suivante: une solution de 4 g de dihydro-5,6 1H, 4H-1,2,5-thiadiazolo [4,3,2-ij] quinoléine dioxyde-2,2 dans 20 $cm^3$ de N,N-diméthyl formamide sec est ajoutée goutte à goutte à une suspension de 0,69 g d'hydrure de sodium (suspension à 80% dans l'huile) dans 20 $cm^3$ de N,N-diméthyl formamide. Après 30 minutes d'agitation, on ajoute une solution de 3,93 g de bromo-1 chloro-4 butane dans 20 $cm^3$ de N,N-diméthyl formamide. Le mélange réactionnel est agité 2 heures à 20°C puis dilué dans un mélange de 200 $cm^3$ d'eau et 200 $cm^3$ d'acétate d'éthyle. La phase organique est lavée à l'eau (3 fois 200 $cm^3$), séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 50 cm) en éluant, sous une pression de 0,7 bar d'azote par un mélange de cyclohexane et d'acétate d'éthyle (80-20 en volumes) et en recueillant des fractions de 125 $cm^3$. Les fractions 3 à 9 sont réunies et concentrées à sec sous pression réduite (2,7 kPa) pour donner 4,5 g de (chloro-4 butyl)-1 dihydro-5,6 1H, 4H-1,2,5-thiadiazolo [4,3,2-ij] quinoléine dioxyde-2,2.

EXEMPLE 7

Une solution de 5,6 g de 6H-dibenzo[c,e] thiazine-1,2 dioxyde-5,5 dans 10 $cm^3$ de N,N-diméthyl formamide sec est ajoutée goutte à goutte à une suspension de 0,72 g d'hydrure de sodium (suspension à 80% dans l'huile) dans 40 $cm^3$ de N,N-diméthyl formamide. Après 30 minutes d'agitation on ajoute une solution de 5,6 g de (chloro-3 propyl)-1 phényl-4 tétrahydro-1,2,3,6 pyridine dans 20 $cm^3$ de N,N-diméthyl formamide. Le mélange réactionnel est chauffé 30 minutes à 100°C et 30 minutes au reflux, puis refroidi et versé dans un mélange de 300 $cm^3$ d'eau et 300 $cm^3$ d'acétate d'éthyle. La phase organique est lavée à l'eau, séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 50 cm) en éluant par du dichlorométhane puis un mélange de dichlorométhane et d'éthanol (98-2 et 96-4 en volumes) et en recueillant des fractions de 250 $cm^3$. Les fractions 8 à 12 sont réunies et concentrées à sec sous pression réduite (2,7 kPa). Le résidu est dissous dans 200 $cm^3$ d'éthanol bouillant. La solution chaude est filtrée et traitée par une solution de 2 g d'acide oxalique dans 20 $cm^3$ d'éthanol. Après refroidissement les cristaux sont essorés, lavés par de l'éthanol et séchés. On obtient 7 g d'oxalate acide de [(phényl-4 tétrahydro-1,2,3,6-pyridyl-1)-3 propyl]-6 6H-dibenzo [c,e]thiazine-1,2 dioxyde-5,5 fondant à 170°C (déc.).

La 6H-dibenzo[c,e]- thiazine-1,2 dioxyde-5,5 peut être préparée selon la méthode décrite par F. ULLMANN et C. GROB, Chem. Ber., 43, 2694 (1910).

EXEMPLE 8

Une solution de 1,7 g de 6H-dibenzo[c,e]thiazine-1,2 dioxyde-5,5 dans 15 $cm^3$ de N,N-diméthyl formamide sec est ajoutée goutte à goutte à une suspension de 0,25 g d'hydrure de sodium (suspension à 80% dans l'huile) dans 5 $cm^3$ de N,N-diméthyl formamide. Après 30 minutes d'agitation on ajoute une solution de 2,1 g de (chloro-3 propyl)-1 (fluoro-4-benzoyl)-4 pipéridine dans 15 $cm^3$ de N,N-diméthyl formamide. Le mélange réactionnel est chauffé 40 minutes à 100°C, puis refroidi et versé dans un mélange de 150 $cm^3$ d'eau et 150 $cm^3$ d'acétate d'éthyle. La phase organique est lavée à l'eau (3 fois 50 $cm^3$), séchée sur sulfate de magnésium et concentrée à sec sous pression réduite (2,7 kPa). Le résidu est chromatographié sur une colonne de gel de silice (granulométrie 0,06-0,2 mm, diamètre 2 cm, hauteur 25 cm) en éluant par un mélange de cyclohexane et d'acétate d'éthyle (50-50 en volumes) et en recueillant des fractions de 30 $cm^3$; les fractions 7 à 16 sont réunies et concentrées à sec sous pression réduite (2,7 kPa). Le résidu est recristallisé dans 40 $cm^3$ d'éthanol; les cristaux obtus sont essorés, lavés par 5 $cm^3$ d'éthanol et séchés. On obtient 1,8 g de [((fluoro-4 pipéridyl-1)-4 pipéridyl-1)-3 propyl]-6 6H-dibenzo[c,e]thiazine-1,2 dioxyde-5,5 fondant à 114°C.

La (chloro-3 propyl)-1 (fluoro-4 benzoyl)-4 pipéridine peut être préparée de la manière suivante: une solution de 3,9 $cm^3$ de bromo-1 chloro-3 propane et de 2,4 g de (fluoro-4 benzoyl)-4 pipéridine dans 20 $cm^3$ d'acétonitrile est agitée 20 heures à 25°C avec 8 g de carbonate de potassium. Le mélange est filtré puis concentré à sec sous pression réduite (2,7 kPa). Le résidu est chromatographié sur une colonne de gel de silice (granulométrie 0,06-0,2 mm, diamètre 2 cm, hauteur 20 cm) en éluant par un mélange de dichlorométhane et d'acétate d'éthyle (60-40 en volumes) et en recueillant des fractions de 15 $cm^3$. Les fractions 9 à 17 sont réunies et concentrées à sec sous pression réduite (2,7 kPa). On obtient 2,1 g de (chloro-3 propyl)-1 (fluoro-4 benzoyl)-4 pipéridine sous forme d'huile.

La (fluoro-4 benzoyl)-4 pipéridine peut être préparée selon la méthode décrite par R. L. DUNCAN et coll. dans J. Med. Chem., 13, 1 (1970).

EXEMPLE 9

Une solution de 2 g de 6H-dibenzo[c,e]thiazine-1,2 dioxyde-5,5 dans 15 $cm^3$ de N,N-diméthyl formamide sec est ajoutée goutte à goutte à une suspension de 0,27 g d'hydrure de sodium (suspension à 80% dans l'huile) dans 5 $cm^3$ de N,N-diméthyl formamide. Après 30 minutes d'agitation on ajoute une solution de 2,2 g de (chloro-3 propyl)-1 (fluoro-4 phényl)-4 pipérazine dans 15 $cm^3$ de N,N-diméthyl formamide. Le mélange réactionnel est chauffé 1 heure et 15 minutes à 100°C, puis refroidi et versé dans un mélange de 200 $cm^3$ d'eau et 180 $cm^3$ d'acétate d'éthyle. La phase organique est lavée à l'eau (3 fois 80 $cm^3$), séchée sur sulfate de magnésium et concentrée à sec sous pression réduite (2,7 kPa). Le résidu est recristallisé dans 60 $cm^3$ d'acétonitrile; les cristaux obtenus sont essorés, lavés par 5 $cm^3$ d'acétonitrile et séchés. On obtient 2,8 g de [((fluoro-4 phényl)-4 pipérazinyl-1)-3 propyl]-6 6H-dibenzo[c,e]thiazine-1,2 dioxyde-5,5 fondant à 163°C.

EXEMPLE 10

Une solution de 8,8 g de 1H, 3H-naphtol[1,8-cd] thiadiazine-1,2,6 dioxyde-2,2 dans 20 $cm^3$ de N,N-diméthyl formamide sec est ajoutée, goutte à goutte à une suspension de 1,2 g d'hydrure de sodium (suspension à 80% dans l'huile) dans 50 $cm^3$ de N,N-diméthyl formamide. Après 15 minutes d'agitation on ajoute une solution de 9,36 g de (chloro-3 propyl)-1 phényl-4 tétrahydro-1,2,3,6 pyridine dans 50 $cm^3$ de N,N-diméthyl formamide. Le mélange réactionnel est chauffé 1 heure à 100°C, puis refroidi et versé dans un mélange de 500 $cm^3$ d'eau et 300 $cm^3$ d'acétate d'éthyle. La phase organique est lavée à l'eau, séchée sur sulfate de magnésium et concentrée à sec sous pression réduite (2,7 kPa). Le résidu est redissous dans 40 $cm^3$ de N,N-diméthyl formamide et la solution diluée par 300 $cm^3$ d'éthanol. Le précipité est essoré, lavé par de l'éthanol et séché puis repris dans 200 $cm^3$ de solution aqueuse 0,1 N de soude dans laquelle il se dissout partiellement. Le mélange est filtré. L'insoluble est essoré, lavé à l'eau et séché. Après recristallisation dans 60 $cm^3$ d'acétate d'éthyle, on obtient 2,38 g de bis-[(phényl-4 tétrahydro-1,2,3,6 pyridyl-1)-3 propyl]-1,3 1H, 3H-naphto[1,8-cd] thiadiazine-1,2,6 dioxyde-2,2 fondant à 140°C.Le filtrat obtenu précédemment est acidifié à pH 4 par de l'acide chlorhydrique 1N. Le précipité est redissous dans 30 $cm^3$ de N,N-diméthyl formamide à 100°C, la solution chaude est filtrée puis diluée par 150 $cm^3$ d'éthanol. Le précipité est essoré, lavé par de l'éthanol (3 fois 50 $cm^3$) et séché. On obtient 0,95 g de [(phényl-4 tétrahydro-1,2,3,6 pyridyl-1)-3 propyl]-1 1H, 3H-naphto[1,8-cd] thiadiazine-1,2,6 dioxyde-2,2 fondant à 240°C.

Le méthyl-3 [(phényl-4 tétrahydro 1,2,3,6 pyridyl-1)-3 propyl]-1 1H, 3H-naphto [1,8-cd] thiadiazine-1,2,6 dioxyde -2,2 peut être préparé de la manière suivante: à une solution de 0,66 g de [(phényl-4 tétrahydro-1,2,3,6 pyridyl-1)-3 propyl]-1 1H, 3H-naphto[1,8-cd]-thiadiazine-1,2,6 dioxyde-2,2 dans 20 $cm^3$ de N,N-diméthyl formamide sec on ajoute 0,105 g d'hydrure de sodium (suspension à 80% dans l'huile). Après 15 minutes d'agitation on ajoute 3 $cm^3$ d'iodure de méthyle. Le mélange réactionnel est agité 1 heure à 20°C, puis dilué dans un mélange de 200 $cm^3$ d'eau et 200 $cm^3$ d'acétate d'éthyle. La phase organique est lavée à l'eau, séchée sur sulfate de magnésium et concentrée à sec sous pression réduite (2,7 kPa). Après chromatographie sur une colonne de gel de silice (granulométrie 0,2-0,063 mm, diamètre 2 cm, hauteur 40 cm) en éluant par des mélanges de cyclohexane et d'acétate d'éthyle (50-50 puis 25-75 en volumes) puis par de l'acétate d'éthyle pur le produit isolé est recristallisé dans 20 $cm^3$ d'acétate d'éthyle. On obtient 0,15 g de méthyl-3 [(phényl-4 tétrahydro-1,2,3,6 pyridyl-1)-3 propyl]-1 1H, 3H-naphto[1,8-cd]thiadiazine-1,2,6 dioxyde-2,2 fondant à 146°C.

Le 1H, 3H-naphto[1,8-cd] thiadiazine-1,2,6 dioxyde-2,2 peut être préparé selon la méthode décrite par C. W. REES et coll.,J. Chem. Soc. 993 (1971).

EXEMPLE 11

Une solution de 1,7 g de dihydro-2,3 naphto[1,2-d]isothiazolone-3 dioxyde-1,1 dans 20 $cm^3$ de N,N-diméthyl formamide sec est ajoutée goutte à goutte à une suspension de 0,27 g d'hydrure de sodium (suspension à 80% dans l'huile) dans 30 $cm^3$ de N,N-diméthyl formamide. Après 30 minutes d'agitation on ajoute une solution de 1,94 g de (chloro-3 propyl)-1 phényl-4 tétrahydro-1,2,3,6 pyridine dans 10 $cm^3$ de N,N-diméthyl formamide. Le mélange réactionnel est chauffé 90 minutes à 80°C, puis refroidi et versé dans un mélange de 200 $cm^3$ d'eau et 200 $cm^3$ d'acétate d'éthyle. La phase organique est lavée à l'eau, séchée sur sulfate de magnésium et concentrée à sec sous pression réduite (2,7 kPa). Le résidu est chromatographié sur une colonne de gel de silice (granulométrie 0,2-0,063 mm, diamètre 3 cm, hauteur 40 cm) en éluant par un mélange de cyclohexane et d'acétate d'éthyle (50-50 en volumes) et en recueillant des fractions de 60 $cm^3$. Les fractions 5 à 12 sont réunies et concentrées à sec sous pression réduite (2,7 kPa). Après recristallisation dans l'acétate d'éthyle on obtient 1,7 g de [(phényl-4 tétrahydro-1,2,3,6-pyridyl-1)-3 propyl]-2 dihydro-2,3 naphto[1,2-d]isothiazolone-3 dioxyde-1,1 fondant à 150°C.

Le dihydro-2,3 naphto[1,2-d]isothiazolone-3 dioxyde-1,1 est préparé selon la méthode décrite par H. P. KAUFMANN et coll., Ber. 6, 1499 (1922).

EXEMPLE 12

Une solution de 3 g de phényl-3 benzisothiazole-1,2 dioxyde-1,1-(3RS) dans 15 $cm^3$ de N,N-diméthyl formamide sec est ajoutée goutte à goutte à une suspension de 0,37 g d'hydrure de sodium (suspension à 80% dans l'huile) dans 10 $cm^3$ de N,N-diméthyl formamide. Après 30 minutes d'agitation on ajoute une solution de 3,2 g de (chloro-3 propyl)-1 (fluoro-4 phényl)-4 pipérazine dans 10 $cm^3$ de N,N-diméthyl formamide. Le mélange réactionnel est chauffé 1 heure à 100°C, puis refroidi et traité par un mélange de 200 $cm^3$ d'eau et 100 $cm^3$ de dichlorométhane. La phase organique est lavée à l'eau (2 fois 80 $cm^3$), séchée sur sulfate de magnésium et concentrée à sec sous pression réduite (2,7 kPa). Le résidu est chromatographié sur une colonne de

gel de silice (granulométrie 0,06-0,2 mm, diamètre 3 cm, hauteur 35 cm) en éluant par un mélange de cyclohexane et d'acétate d'éthyle (60-40 en volumes) et en recueillant des fractions de 30 $cm^3$. Les fractions 13 à 24 sont réunies et concentrées à sec sous pression réduite (2,7 kPa). Le résidu est dissous dans 20 $cm^3$ d'éthanol bouillant, puis la solution chaude est traitée par une solution de 0,8 g d'acide fumarique dans 15 $cm^3$ d'eau. Les cristaux obtenus sont essorés, lavés par 5 $cm^3$ d'éthanol, par 10 $cm^3$ d'éther et séchés. On obtient 2,6 g de sesquifumarate de [((fluoro--4 phényl)-4 pipérazinyl-1)-3 propyl]-2 phényl-3 benzisothiazole-1,2 dioxyde-1,1-(3RS) fondant à 182 °C.

Le phényl-3 benzisothiazole-1,2 dioxide-1,1 peut être préparé de la manière suivante: à une solution de 80 $cm^3$ d'acide sulfurique concentré refroidie à 0°C, on ajoute 10 g de N-tert-butyl (α-hydroxy benzyl)-2 benzènesulfonamide. Le mélange est ensuite agité 1 heure à 25°C, puis versé dans 800 $cm^3$ d'eau glacée. Après 1 heure d'agitation, le précipité est essoré puis repris par 100 $cm^3$ de dichlorométhane. La solution organique est lavée à l'eau (2 fois 50 $cm^3$), séchée sur sulfate de magnésium et concentrée à sec sous pression réduite (2,7 kPa). On obtient 7,1 g de phényl-3 benzisothiazole-1,2 dioxide-1,1 fondant à 118°C.

Le N-tert-butyl (α-hydroxy benzyl)-2 benzènesulfonamide peut être préparé de la manière suivante: à une solution de 8,5 g de N-tert-butyl benzènesulfonamide à dans 100 $cm^3$ de tétrahydrofuranne sec refroidie à 0 °C, on ajoute 64 $cm^3$ d'une solution de N-butyllithium 1,6 M dans l'hexane. Après une heure d'agitation, on ajoute une solution de 6,5 $cm^3$ de benzaldéhyde dans 30 $cm^3$ de tétrahydrofuranne sec, puis on poursuit l'agitation 2 heures à 0°C. Le mélange est traité par 30 $cm^3$ d'acide chlorhydrique 2 N et extrait par 100 $cm^3$ d'acétate d'éthyle; la solution organique est lavée par 50 $cm^3$ d'eau, séchée sur sulfate de magnésium et concentrée à sec sous pression réduite (2,7 kPa). Le résidu solide est lavé par 50 $cm^3$ d'oxyde d'isopropyle, essoré et séché. On obtient 11,7 g de N-tert-butyl (α-hydroxy benzyl)-2 benzènesulfonamide fondant à 160°C.

Le N-tert-butyl benzènesulfonamide peut être préparé par la méthode décrite par J.G. LOMBARDINO, J. Org. Chem., 36, 1843, (1971).

EXEMPLE 13

Une solution de 2,45 g de phényl-3 benzisothiazole-1,2 dioxyde-1,1-(3RS) dans 10 $cm^3$ de N,N-diméthyl formamide sec est ajoutée goutte à goutte à une suspension de 0,3 g d'hydrure de sodium (suspension à 80% dans l'huile) dans 5 $cm^3$ de N,N-diméthyl formamide. Après 30 minutes d'agitation on ajoute une solution de 2,4 g de (chloro-3 propyl)-1 phényl-4 pipéridine dans 5 $cm^3$ de N,N-diméthyl formamide. Le mélange réactionnel est chauffé 1 heure et 30 minutes à 100°C, puis refroidi et concentré à sec sous pression réduite (0,1 kPa). Le résidu est chromatographié sur une colonne de gel de silice (granulométrie 0,06-0,2 mm, diamètre 3 cm, hauteur 30 cm) en éluant par un mélange de cyclohexane et d'acétate d'éthyle (50-50 en volumes) et en recueillant des fractions de 50 $cm^3$. Les fractions 12 à 24 sont réunies et concentrées à sec sous pression réduite (2,7 kPa). Le résidu est dissous dans 10 $cm^3$ d'éthanol bouillant, puis la solution chaude est traitée par une solution de 0,37 g d'acide fumarique dans 5 $cm^3$ d'eau. Les cristaux obtenus sont essorés, lavés par 5 $cm^3$ d'éthanol, par 10 $cm^3$ d'éther et séchés. On obtient 1,6 g de fumarate acide de phényl-3 [(phényl-4 pipéridyl-1)-3 propy]-2 benzisothiazole-1,2 dioxyde-1,1 -(3RS) fondant à 191°C.

La (chloro-3 propyl)-1 phényl-4 pipéridine peut être obtenue de la manière suivante: une solution de 8 g de phényl-4 pipéridine et de 20 $cm^3$ de bromo-1 chloro-3 propane dans 80 $cm^3$ d'acétonitrile est agitée 24 heures à 25°C avec 28 g de carbonate de potassium. Le mélange est filtré puis concentré à sec sous pression réduite (2,7 kPa). Le résidu est chromatographié sur une colonne de gel de silice (granulométrie 0,06-0,2 mm, diamètre 3 cm, hauteur 25 cm) en éluant par un mélange de cyclohexane et d'acétate d'éthyle (50-50 en volumes) et en recueillant des fractions de 60 $cm^3$. Les fractions 6 à 10 sont réunies et concentrées à sec sous pression réduite (2,7 kPa). On obtient 8 g de (chloro-3 propyl)-1 phényl-4 pipéridine sous forme d'huile.

EXEMPLE 14

Une solution de 6,6 g de N-(naphtyl-1) méthanesulfonamide dans 20 $cm^3$ de N,N-diméthyl formamide sec est ajoutée goutte à goutte à une suspension de 0,9 g d'hydrure de sodium (suspension à 80% dans l'huile) dans 50 $cm^3$ de N,N-diméthyl formamide. Après agitation pendant 15 minutes on ajoute une solution de 7,2 g de (chloro-3 propyl)-1 (fluoro-4 phényl)-4 pipérazine dans 20 $cm^3$ de N,N- diméthyl formamide. Le mélange réactionnel est chauffé 2 heures et 30 minutes à 110°C, puis refroidi et versé dans un mélange de 300 $cm^3$ d'eau et 500 $cm^3$ d'acétate d'éthyle. La phase organique est lavée à l'eau, diluée par 50 $cm^3$ de dichlorométhane, séchée sur sulfate de magnésium et concentrée à sec sous pression réduite (2,7 kPA) jusqu'à 50 $cm^3$. Les cristaux sont filtrés, lavés par de l'acétate d'éthyle (3 fois 50 $cm^3$) puis par de l'oxyde d'isopropyle (2 fois 30 $cm^3$) et séchés. Après recristallisation dans 300 $cm^3$ d'acétate d'éthyle, on obtient 4,84 g de N-[((fluoro-4-phényl)-4 pipérazinyl-1)-3 propyl] N-(naphtyl-1) méthanesulfonamide fondant à 170°C.

EXEMPLE 15

Une solution de 6,6 g de N-(naphtyl-1) méthanesulfonamide dans 10 $cm^3$ de N,N-diméthyl formamide sec est ajoutée goutte à goutte à une suspension de 0,9 g d'hydrure de sodium (suspension à 80% dans l'huile) dans 50 $cm^3$ de N,N-diméthyl formamide. Après 30 minutes d'agitation on ajoute une solution de 7,2 g de (chloro-3 propyl)-1 phényl-4 tétrahydro-1,2,3,6 pyridine dans 20 $cm^3$ de N,N-diméthyl formamide. Le mélange réactionnel est chauffé 1 heure au reflux, puis refroidi et versé dans un mélange de 300 $cm^3$ d'eau et 300 $cm^3$ d'acétate d'éthyle. La phase organique est lavée à l'eau, séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 60 cm) en éluant par du dichlorométhane puis par un mélange de dichlorométhane et d'éthanol (98-2 et 96-4 en volumes) et en recueillant des fractions de 250 $cm^3$. Les fractions 11 à 15 sont réunies et concentrées à sec sous pression réduite (2,7 kPa). Après recristallisation dans 80 $cm^3$ d'acétate d'éthyle on obtient 4,57 g de N-[(phényl-4 tétrahydro-1,2,3,6 pyridyl-1)-3 propyl] N-(naphtyl-1) methanesulfonamide fondant à 162°C.

EXEMPLE 16

Une solution de 2,34 g de N-phényl benzènesulfonamide dans 10 $cm^3$ de N,N-diméthyl formamide est ajoutée goutte à goutte à une suspension de 0,24 g d'hydrure de sodium (suspension à 80% dans l'huile) dans 50 $cm^3$ de N,N-diméthyl formamide. Après 15 minutes d'agitation on ajoute une solution de 2,34 g de (chloro-3 propyl)-1 phényl-4 tétrahydro-1,2,3,6 pyridine dans 20 $cm^3$ de N,N- diméthyl formamide. Le mélange réactionnel est chauffé 1 heure à 140 °C, puis refroidi et versé dans un mélange de 200 $cm^3$ d'eau et 200 $cm^3$ d'acétate d'éthyle. La phase organique est lavée à l'eau, séchée sur sulfate de magnésium et concentrée à sec sous pression réduite (2,7 kPa). Le résidu est chromatographié sur une colonne de gel de silice (granulométrie 0,2-0,063 mm, diamètre 4 cm, hauteur 40 cm) en éluant par du dichlométhane puis par un mélange de dichlométhane et d'éthanol (98-2 et 96-4 en volumes) et en recueillant des fractions de 250 $cm^3$. Les fractions 5 à 9 sont réunies et concentrées à sec sous pression réduite (2,7 kPa). Après recristallisation dans 150 $cm^3$ d'oxyde d'isopropyle on obtient 2, 4 g de N-[(phényl-4 tétrahydro-1,2,3,6 pyridyl)-3 propyl] N-phényl benzènesulfonamide fondant à 112°C.

EXEMPLE 17

A un mélange de 1,3 g d'hydrure de sodium en dispersion à 50% dans l'huile de vaseline et de 10 $cm^3$ de diméthylformamide, sous courant d'argon, on coule, en 30 minutes, une solution de 4,5 g de benzo[c,d] indolone-2 dans 25 $cm^3$ de diméthylformamide. Le milieu réactionnel est agité 30 minutes à 100°C, puis refroidi à une température voisine de 20°C. On ajoute ensuite, en 10 minutes, 9,3 g de (bromo-3 propyl)-1 phényl-4 tétrahydro-1,2,3,6 pyridine dans 20 $cm^3$ de diméthylformamide. Le mélange réactionnel est agité 2 heures au reflux puis refroidi à une température proche de 20°C. L'huile résiduelle est concentrée à sec à 40°C sous pression réduite (20 mm de mercure; 2,7 kPa) puis purifiée par flash-chromatographie sur colonne de silice, sous courant d'argon, à moyenne pression (0,5-1,5 bar), avec de l'acétate d'éthyle comme éluant. On obtient 7,4 g de N-[(phényl-4 tétrahydro-1,2,3,6 pyridyl)-3 propyl] benzo[c,d] indolone-2 sous forme d'une huile jaune (oxalate acide; point de fusion: 138°C).

La (bromo-3 propyl)-1 phényl-4 tétrahydro-1,2,3,6 pyridine peut être préparée de la manière suivante: à 8,7 g d'(hydroxy-3 propyl)-1 phényl-4 tétrahydro-1,2,3,6 pyridine dans 100 $cm^3$ de toluène, on coule 2,7 $cm^3$ de tribromure de phosphore. Le mélange est chauffé 2 heures à reflux puis refroidi à une température proche de 20°C. le précipité formé est filtré sur verre fritté puis repris par 250 $cm^3$ de dichlorométhane et 150 $cm^3$ d'eau distillé. La phase organique est décantée, séchée sur du sulfate de magnésium anhydre, filtrée et concentrée à sec à 40°C sous pression réduite (20 mm de mercure; 2,7 kPa). On obtient 14 g de (bromo-3 propyl)-1 phényl-4 tétrahydro-1,2,3,6 pyridine, à l'état de sel de bromhydrate (point de fusion: 185°C), utilisé à l'état brut dans les synthèses ultérieures.

L'(hydroxy-3 propyl)-1 phényl-4 tétrahydro-1,2,3,6 pyridine peut être préparée de la manière suivante: 25 $cm^3$ de bromo-3 propanol, 73,4 $cm^3$ de triéthylamine et 51,5 g de chlorhydrate de phényl-4 tétrahydro-1,2,3,6 pyridine dans 700 $cm^3$ de toluène sont chauffés à ébullition pendant 16 heures. Le mélange est ensuite refroidi à une température voisine de 20°C puis concentrée à sec à 40°C sous pression réduite (20 mm de mercure; 2,7 kPa). On obtient 42 g d'(hydroxy-3 propyl)-1 phényl-4 tétrahydro-1,2,3,6 pyridine, sous forme d'une huile brune qui cristallise (point de fusion < 40°C), utilisée à l'état brut dans les synthèses ultérieures.

Les médicaments selon l'invention sont constitués par un composé de formule (I) sous forme libre ou sous forme d'un sel d'addition avec un acide pharmaceutiquement acceptable, à l'état pur ou sous forme d'une

composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvant organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles pour le traitement des affections où la sérotonine est impliquée et notamment les affections du système nerveux central, du système cardiovasculaire et les troubles intestinaux. Ils sont, en particulier, utiles pour le traitement de l'anxiété, des troubles du sommeil, de la dépression, des psychoses et notamment de la schizophrénie, de la migraine, de l'asthme, de l'hypertension et de l'urticaire, comme analgésiques et comme inhibiteurs de l'agrégation plaquettaire.

Les doses dépendent de l'effet recherché, de la durée du traitement et de la voie d'administration utilisée ; elles sont généralement comprises entre 10 et 300 mg par jour par voie orale pour un adulte avec des doses unitaires allant de 5 à 150 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

- [(phényl-4 tétrahydro-1,2,3,6 pyridyl-1)-3 propyl]-1 dihydro-5,6 1H, 4H-1,2,5 thiadiazolo [4,3,2-ij] quinoléine dioxyde-2,2 50 mg
- cellulose 18 mg
- lactose 55 mg
- silice colloïdale 1 mg
- carbaxyméthylamidon sodique 10 mg
- talc 10 mg
- stéarate de magnésium 1 mg

EXEMPLE B

On prépare, selon la technique habituelle, des comprimés dosés à 50 mg de produit actif ayant la composition habituelle :

- bis-[(phényl-4 tétrahydro-1,2,3,6 pyridyl-1)-3 propyl]-1,3 1H,3H-naphto [1,8-cd] thiadiazine-1,2,6 dioxyde-2,2 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (71-3, 5-24, 5) q.s.p. 1 comprimé pelliculé terminé à 245 mg

EXEMPLE C

On prépare une solution injectable contenant 10 mg de produit actif ayant la composition suivante :

- [((fluoro-4 phényl)-4 pipérazinyl)-3 propyl]-1 dihydro-5,6 1H, 4H-1,2,5 thiadiazolo [4,3,2-ij] quinoléine dioxyde 2,2 10 mg
- acide benzoïque 80 mg
- alcool benzylique 0,06 $cm^3$
- benzoate de sodium 80 mg
- ethanol à 95 % 0,4 $cm^3$
- hydroxyde de sodium 24 mg
- propylène glycol 1,6 $cm^3$
- eau q.s.p 4 $cm^3$

## Revendications

### Revendications pour les Etats contractants suivants : AT, BE, CH, DE, DK, FR, GB, IT, LI, LU, NL, SE

**1.** Composés de formule:

$$R_2 - \underset{\displaystyle R_3}{\underset{|}{N}} - (CH_2)_n - R_1 \qquad (I)$$

dans laquelle

- $R_1$ représente
  - . un radical tétrahydro-1,2,3,6 pyridyl-1 substitué en position -4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
  - . un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1,2 yl-3, (d) un radical benzisoxazol-1,2 yl-3 ou (e) un radical pyridyl-2,
  - . un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl-1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3, (j) un radical indolyl-3 substitué sur l'atome d'azote par un radical alkyle ou alkylcarbonyle et/ou en position -5 par un atome de chlore ou de fluor ou (k) un radical (hydroxy-5 indolyl)-3,
- $R_2$ représente un radical $SO_2R_4$ dans lequel $R_4$ représente un radical alkyle ou phényle,

- $R_3$ représente un radical phényle ou naphtyle,
- ou bien $R_2$ et $R_3$ forment ensemble avec l'atome d'azote auquel ils sont attachés un cycle choisi parmi les formules:

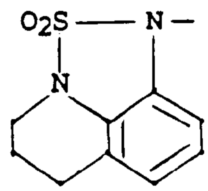

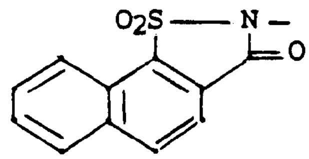

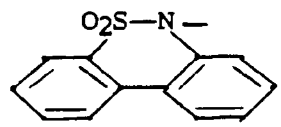

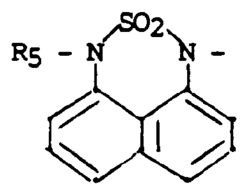

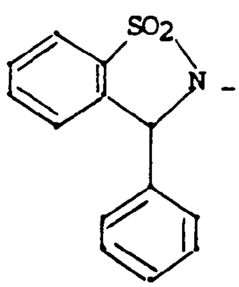

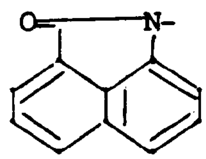

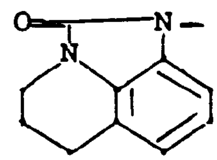

- $R_5$ représente un radical alkyle ou une chaîne $-(CH_2)_n-R_1$,
- n est égal à 2, 3 ou 4,

et leurs sels avec des acides minéraux ou organiques, étant entendu que les radicaux alkyle et alcoxy ou les portions alkyle contiennent 1 à 4 atomes de carbone en chaîne droite ou ramifiée.

**2.** Composés de formule (I) selon la revendication 1 pour lesquels $R_1$ représente un radical phényl-4 tétrahydro-1,2,3,6 pyridyl-1, phényl-4 pipéridino, (fluoro-4 benzoyl)-4 pipéridino ou phényl-4 pipérazinyl-1 dont le noyau phényle est éventuellement substitué par un atome d'halogène ou un radical hydroxy, $R_2$, $R_3$ et n ont les mêmes significations que dans la revendication 1, ainsi que leurs sels avec des acides minéraux ou organiques.

**3.** Composés de formule (I) selon les revendications 1 ou 2 pour lesquels n est égal à 3.

**4.** Composés de formule (I) selon l'une des revendications 1 à 3 pour lesquels $R_2$ et $R_3$ forment avec l'atome d'azote auquel ils sont rattachés un cycle dihydro-5,6 1H, 4H-1,2,5-thiadiazolo [4,3,2-ij] quinoléine dioxyde-2,2 ou phényl-3 benzothiazole-1,2 dioxyde-1,1.

**5.** Procédé de préparation des composés de formule (I) selon la revendication 1, à l'exception de ceux pour lequels $R_1$ représente un radical aminophényl-4 pipérazinyl-1 caractérisé en ce que l'on fait réagir un dérivé de formule :

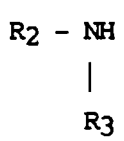

(II)

dans laquelle $R_2$ et $R_3$ ont les mêmes significations que dans la revendication 1, sur un dérivé halogéné de formule :

$$Hal - (CH_2)_n - R_1 \qquad (III)$$

dans laquelle Hal représente un atome d'halogène, n a les mêmes significations que dans la revendication 1 et $R_1$ a les mêmes significations que ci-dessus, isole le produit et le transforme éventuellement en sel d'addition avec un acide minéral ou organique.

**6.** Procédé de préparation des composés de formule (I) selon la revendication 1, à l'exception de ceux pour lesquels $R_1$ représente un radical aminophényl-4 pipérazinyl-1, caractérisé en ce que l'on fait réagir un dérivé de formule :

$$R_2 - \underset{\displaystyle R_3}{\underset{|}{N}} - (CH_2)_n - Hal \qquad (VI)$$

sur un dérivé de formule :

$$HR_1 \qquad (IV)$$

dans lesquelles $R_1$ a les mêmes significations que ci-dessus, $R_2$, $R_3$ et n ont les mêmes significations que dans la revendication 1 et Hal représente un atome d'halogène, isole le produit et le transforme éventuellement en sel d'addition avec un acide minéral ou organique.

**7.** Procédé de préparation des composés de formule (I) selon la revendication 1 pour lesquels $R_1$ représente un radical aminophényl-4 pipérazinyl-1 caractérisé en ce que l'on réduit le composé de formule (I) correspondant lequel $R_1$ représente un radical nitrophényl-4 pipérazinyl-1, isole le produit et le transforme éventuellement en sel d'addition avec un acide minéral ou organique.

**8.** Médicaments caractérisés en ce qu'ils contiennent en tant que principe actif au moins un composé de formule (I) selon la revendication 1.

**9.** Médicaments selon la revendication 8 pour le traitement de maladies où la sérotonine est impliquée.

**Revendications pour les Etats contractants suivants : ES, GR**

**1.** Procédé de préparation des composés de formule :

$$R_2 - \underset{\displaystyle R_3}{\underset{|}{N}} - (CH_2)_n - R_1 \qquad (I)$$

dans laquelle

- $R_1$ représente
  . un radical tétrahydro-1,2,3,6 pyridyl-1 substitué en position -4 par (a) un radical phényle, (b) un radical phényle substitué par un atome d'halogène ou un radical alkyle, hydroxy ou alcoxy, (c) un radical indolyl-3, (d) un radical indolyl-3 substitué sur l'atome d'azote par un radical alkyle ou alkylcarbonyle et/ou en position -5 par un atome de chlore ou de fluor ou (e) un radical (hydroxy-5 indolyl)-3,
  . un radical pipérazinyl-1 substitué en position -4 par (a) un radical phényle, (b) un radical phényle substitué par un radical alcoxy, alkyle, hydroxy, nitro, amino ou un atome d'halogène, (c) un radical benzisothiazol-1,2 yl-3, (d) un radical benzisoxazol-1,2 yl-3 ou (e) un radical pyridyl-2,
  . un radical pipéridino substitué en position -4 par (a) un radical phényle, (b) un radical phényle substitué par un atome d'halogène ou un radical hydroxy, alkyle ou alcoxy, (c) deux radicaux phényle, (d) un radical bis (fluoro-4 phényl) méthylène, (e) un radical fluoro-4 benzoyle, (f) un radical oxo-2 benzimidazolinyl-1, (g) un radical oxo-2 benzimidazolinyl-1 substitué en position -3 par un radical alkylcarbonyle ou benzoyle, (h) un radical hydroxy et un radical phényle éventuellement substitué par un radical alkyle, alcoxy, hydroxy ou un atome d'halogène, (i) un radical indolyl-3,

(j) un radical indolyl-3 substitué sur l'atome d'azote par un radical alkyle ou alkylcarbonyle et/ou en position -5 par un atome de chlore ou de fluor ou (k) un radical (hydroxy-5 indolyl)-3,

- $R_2$ représente un radical $SO_2R_4$ dans lequel $R_4$ représente un radical alkyle ou phényle,
- $R_3$ représente un radical phényle ou naphtyle,
- ou bien $R_2$ et $R_3$ forment ensemble avec l'atome d'azote auquel ils sont attachés un cycle choisi parmi les formules :

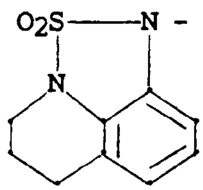

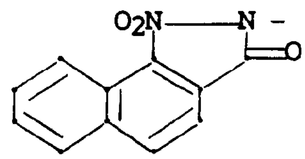

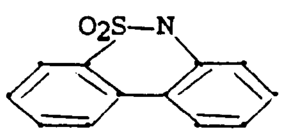

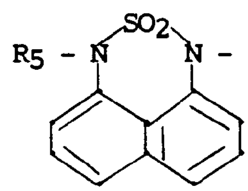

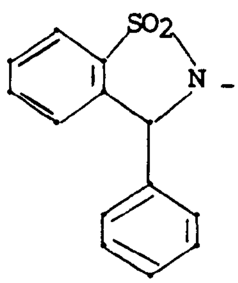

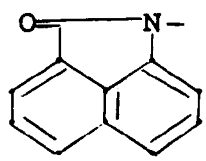

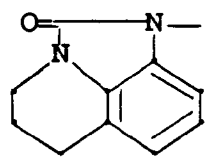

- $R_5$ représente un radical alkyle ou une chaîne $-(CH_2)_n-R_1$,
- n est égal à 2, 3 ou 4 et leurs sels avec des acides minéraux ou organiques, caractérisé en ce que

A - pour la préparation des composés de formule (I) à l'exception des composés pour lesquels $R_1$ représente un radical aminophényl-4 pipérazinyl-1, on fait réagir un dérivé de formule :

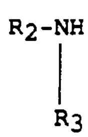

(II)

dans laquelle $R_2$ et $R_3$ ont les mêmes significations que ci-dessus, sur un dérivé halogéné de formule :

$$Hal-(CH_2)_n-R_1 \quad (III)$$

dans laquelle Hal représente un atome d'halogène, n a les mêmes significations que dans la formule (I) et $R_1$ a les mêmes significations que précédemment, isole le produit et le transforme éventuellement en sel d'addition avec un acide minéral ou organique,

B - pour la préparation des composés de formule (I) à l'exception de ceux pour lesquels $R_1$ représente un radical aminophényl-4 pipérazinyl-1 on fait réagir un dérivé de formule :

$$R_2-\underset{\displaystyle R_3}{\underset{|}{N}}-(CH_2)_n-Hal \qquad (VI)$$

dans laquelle n, $R_2$ et $R_3$ ont les mêmes significations que dans la formule (I) et Hal représente un atome d'halogène sur un dérivé de formule :

$$HR_1 \qquad (VI)$$

dans laquelle $R_1$ a les mêmes significations que ci-dessus, isole le produit et le transforme éventuellement en sel d'addition avec un acide minéral ou organique.

C - pour la préparation des composés de formule (I) pour lesquels $R_1$ représente un radical aminophényl-4 pipérazinyl-1 on réduit le composé de formule (I) correspondant pour lequel $R_1$ représente un radical nitrophényl-4 pipérazinyl-1, isole le produit et le transforme éventuellement en sel d'addition avec un acide minéral ou organique.

2. Procédé selon la revendication 1 pour la préparation des composés de formule (I) pour lesquels $R_1$ représente un radical phényl-4 tétrahydro-1,2,3,6 pyridyl-1, phényl-4 pipéridino, (fluoro-4 benzoyl)-4 pipéridino ou phényl-4 pipérazinyl-1 dont le noyau phényle est éventuellement substitué par un atome d'halogène ou un radical hydroxy, $R_2$, $R_3$ et n ont les mêmes significations que dans la revendication 1, ainsi que leurs sels avec des acides minéraux ou organiques.

3. Procédé selon la revendication 1 pour la préparation des composés de formule (I) pour lesquels n est égal à 3.

4. Procédé selon la revendication 1 pour la préparation des composés de formule (I) pour lesquels $R_2$ et $R_3$ forment avec l'atome d'azote auquel ils sont rattachés un cycle dihydro-5,6 1H, 4H-1,2,5-thiadiazolo [4,3,2-ij] quinoléine dioxyde-2,2 ou phényl-3 benzothiazole-1,2 dioxyde-1,1.

## Patentansprüche

### Patentansprüche für folgende Vertragsstaaten : AT, BE, CH, DE, DK, FR, GB, IT, LI, LU, NL, SE

1. Verbindungen der Formel (I)

$$R_2-\underset{\displaystyle R_3}{\underset{|}{N}}-(CH_2)_n-R_1 \qquad (I)$$

in der

- $R_1$ darstellt
  - . einen 1,2,3,6-Tetrahydro-1-pyridylrest, substituiert in 4-Stellung durch (a) einen Phenylrest, (b) einen Phenylrest. substituiert durch ein Halogenatom oder einen Alkyl-, Hydroxy- oder Alkoxyrest, (c) einen 3-Indolylrest, (d) einen 3-Indolylrest, substituiert am Stickstoffatom durch einen Alkyl- oder Alkylcarbonylrest und/oder in 5-Stellung durch ein Chloratom oder Fluoratom oder (e) einen 3-(5-Hydroxy-indolyl)-Rest.
  - . einen 1-Piperazinylrest, substituiert in 4-Stellung durch (a) einen Phenylrest, (b) einen Phenylrest, substituiert durch einen Alkoxy-, Alkyl-, Hydroxy-, Nitro-, Aminorest oder ein Halogenatom, (c) einen 1,2-Benzisothiazol-3-yl-Rest, (d) einen 1,2-Benzisoxazol-3-yl-Rest oder (e) einen 2-Pyridylrest,
  - . einen Piperidinorest, substituiert in 4-Stellung durch (a) einen Phenylrest, (b) einen Phenylrest, substituiert durch ein Halogenatom oder einen Hydroxy-, Alkyl- oder Alkoxyrest, (c) zwei Phenylreste, (d) einen Bis-(4-Fluorphenyl)-methylen-Rest, (e) einen 4-Fluorbenzoyl-Rest, (f) einen 2-Oxo-1-benzimidazolinyl-Rest, (g) einen 2-Oxo-1-benzimidazolinyl-Rest, substituiert in 3-Stellung durch einen Alkylcarbonyl- oder Benzoylrest, (h) einen Hydroxyrest und einen Phenylrest, gegebenenfalls substituiert durch einen Alkyl-, Alkoxy-, Hydroxyrest oder ein Halogenatom, (i) einen

3-Indolylrest, (j) einen 3-Indolylrest, substituiert am Stickstoffatom durch einen Alkyl- oder Alkylcarbonylrest und/oder in 5-Stellung durch ein Chloratom oder Fluoratom, oder (k) einen 3-(5-Hydroxy-indolyl)-Rest,

- $R_2$ einen $SO_2R_4$-Rest darstellt, worin $R_4$ ein Alkyl- oder Phenylrest ist,
- $R_3$ einen Phenyl- oder Naphthylrest bedeutet,
- oder auch $R_2$ und $R_3$ zusammen mit dem Stickstoffatom, an das sie gebunden sind, einen Cyclus darstellen, ausgewählt unter den folgenden Formeln:

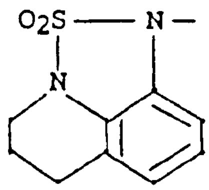

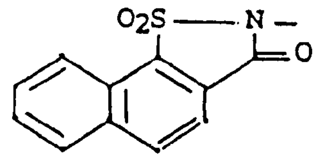

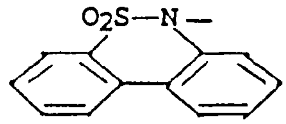

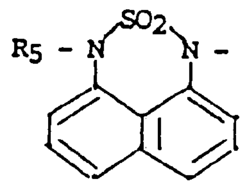

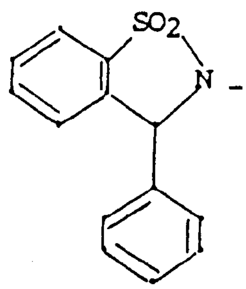

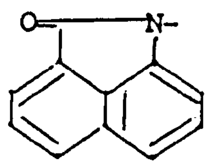

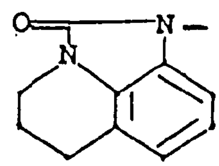

- $R_5$ einen Alkylrest oder eine Kette $-(CH_2)n-R_1$ darstellt,
- n gleich 2, 3 oder 4 ist,

und ihre Salze mit Mineralsäuren oder organischen Säuren. mit der Maßgabe, daß die Alkyl- oder Alkoxyreste, oder die Alkylteile 1 bis 4 Kohlenstoffatome in gerader oder verzweigter Kette enthalten.

2. Verbindungen der Formel (I) nach Anspruch 1, in der $R_1$ einen 4-Phenyl-1,2,3,6-tetrahydro-1-pyridyl-, 4-Phenyl-piperidino-, 4-(4-Fluorbenzoyl)-piperidino- oder 4-Phenyl-1-piperazinyl-Rest darstellt, deren Phenylkern gegebenenfalls durch ein Halogenatom oder einen Hydroxyrest substituiert ist, $R_2$. $R_3$ und n die gleichen Bedeutungen wie in Anspruch 1 besitzen, sowie ihre Salze mit Mineralsäuren oder organischen Säuren.

3. Verbindungen der Formel (I) nach Anspruch 1 oder 2, in der n gleich 3 ist.

4. Verbindungen der Formel (I) nach einem der Ansprüche 1 bis 3, in der $R_2$ und $R_3$ zusammen mit dem Stickstoffatom. an das sie gebunden sind, einen 5,6-Dihydro-1H,4H-1,2,5-thiadiazolo[4,3,2-ij]-chinolin-2,2-dioxid- oder 3-Phenyl-1,2-benzothiazol-1,1-dioxid-Ring bilden.

5. Verfahren zur Herstellung der Verbindungen der Formel (I) nach Anspruch 1, mit Ausnahme der Verbindungen, in denen $R_1$ einen 4-Aminophenyl-1-piperazinyl-Rest darstellt, dadurch gekennzeichnet, daß man ein Derivat der Formel (II)

$$R_2 - NH - R_3 \qquad (II)$$

in der $R_2$ und $R_3$ die gleichen Bedeutungen wie in Anspruch 1 besitzen. mit einem halogenierten Derivat der Formel (III)

$$Hal - (CH_2)_n - R_1 \qquad (III)$$

zur Reaktion bringt, in der Hal ein Halogenatom darstellt, n die gleichen Bedeutungen wie in Anspruch 1 besitzt und $R_1$ die gleichen Bedeutungen wie vorstehend aufweist, daß man das Produkt isoliert und gegebenenfalls in ein Additionssalz mit einer Mineralsäure oder organischen Säure umwandelt.

6. Verfahren zur Herstellung der Verbindungen der Formel (I) nach Anspruch 1, mit Ausnahme der Verbindungen, in denen $R_1$ einen 4-Aminophenyl-1-piperazinyl-Rest darstellt, dadurch gekennzeichnet, daß man ein Derivat der Formel (VI)

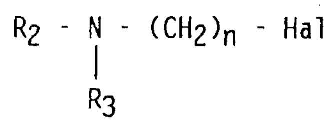

(VI)

mit einem Derivat der Formel (IV)

$$HR_1 \qquad (IV)$$

zur Reaktion bringt, in denen $R_1$ die gleichen Bedeutungen wie vorstehend aufweist, $R_2$, $R_3$ und n die gleichen Bedeutungen wie in Anspruch 1 besitzt und Hal ein Halogenatom darstellt, daß man das Produkt isoliert und gegebenenfalls in ein Additionssalz mit einer Mineralsäure oder organischen Säure umwandelt.

7. Verfahren zur Herstellung der Verbindungen der Formel (I) nach Anspruch 1, in der $R_1$ einen 4-Aminophenyl-1-piperazinyl-Rest darstellt, dadurch gekennzeichnet, daß man die Verbindung der Formel (I), in der $R_1$ einen 4-Nitrophenyl-1-piperazinyl-Rest bedeutet, reduziert und man das Produkt isoliert und gegebenenfalls in ein Additionssalz mit einer Mineralsäure oder organischen Säure umwandelt.

8. Medikamente, dadurch gekennzeichnet, daß sie als Wirkstoff mindestens eine Verbindung der Formel (I) nach Anspruch 1 enthalten.

9. Medikamente nach Anspruch 8 zur Behandlung von Erkrankungen bei denen Serotonin einbezogen ist.

**Patentansprüche für folgende Vertragsstaaten : ES, GR**

1. Verfahren zur Herstellung von Verbindungen der Formel (I)

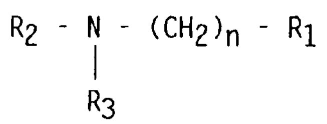

(I)

in der

- $R_1$ darstellt
  . einen 1,2,3,6-Tetrahydro-1-pyridylrest, substituiert in 4-Stellung durch (a) einen Phenylrest, (b) einen Phenylrest, substituiert durch ein Halogenatom oder einen Alkyl-. Hydroxy- oder Alkoxyrest. (c) einen 3-Indolylrest, (d) einen 3-Indolylrest, substituiert am Stickstoffatom durch einen Alkyl- oder Alkylcarbonylrest und/oder in 5-Stellung durch ein Chloratom oder Fluoratom oder (e) einen 3-(5-Hydroxy-indolyl)-Rest,
  . einen 1-Piperazinylrest, substituiert in 4-Stellung durch (a) einen Phenylrest, (b) einen Phenylrest, substituiert durch einen Alkoxy-, Alkyl-, Hydroxy-, Nitro-, Aminorest oder ein Halogenatom, (c) einen 1,2-Benzisothiazol-3-yl-Rest, (d) einen 1,2-Benzisoxazol-3-yl-Rest oder (e) einen 2-Pyridylrest,
  . einen Piperidinorest, substituiert in 4-Stellung durch (a) einen Phenylrest. (b) einen Phenylrest. substituiert durch ein Halogenatom oder einen Hydroxy-, Alkyl- oder Alkoxyrest, (c) zwei Phenylreste, (d) einen Bis-(4-Fluorphenyl)-methylen-Rest, (e) einen 4-Fluorbenzoyl-Rest, (f) einen 2-Oxo-1-benzimidazolinyl-Rest, (g) einen 2-Oxo-1-benzimidazolinyl-Rest, substituiert in 3-Stellung durch einen Alkylcarbonyl- oder Benzoylrest, (h) einen Hydroxyrest und einen Phenylrest, gegebenenfalls substituiert durch einen Alkyl-, Alkoxy-, Hydroxyrest oder ein Halogenatom, (i) einen

3-Indolylrest, (j) einen 3-Indolylrest, substituiert am Stickstoffatom durch einen Alkyl- oder Alkylcarbonylrest und/oder in 5-Stellung durch ein Chloratom oder Fluoratom, oder (k) einen 3-(5-Hydroxy-indolyl)-Rest.

- $R_2$ einen $SO_2R_4$-Rest darstellt, worin $R_4$ ein Alkyl- oder Phenylrest ist,
- $R_3$ einen Phenyl- oder Naphthylrest bedeutet.
- oder auch $R_2$ und $R_3$ zusammen mit dem Stickstoffatom, an das sie gebunden sind, einen Cyclus darstellen, ausgewählt unter den folgenden Formeln:

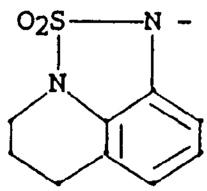

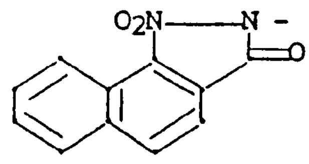

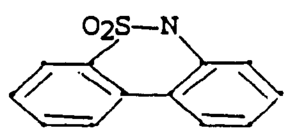

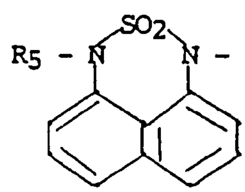

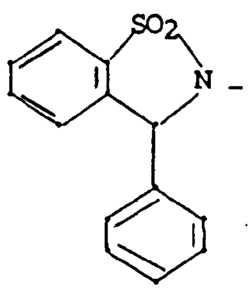

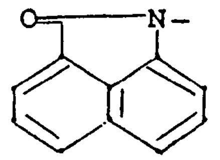

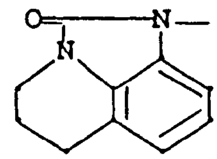

- $R_5$ einen Alkylrest oder eine Kette $-(CH_2)_n-R_1$ darstellt,
- n gleich 2, 3 oder 4 ist,

und ihre Salze mit Mineralsäuren oder organischen Säuren, dadurch gekennzeichnet, daß man

A - zur Herstellung der Verbindungen der Formel (I), mit Ausnahme der Verbindungen, in denen $R_1$ einen 4-Aminophenyl-1-piperazinyl-Rest darstellt, ein Derivat der Formel (II)

$$R_2 - NH - R_3 \qquad (II)$$

in der $R_2$ und $R_3$ die gleichen Bedeutungen wie vorstehend besitzen. mit einem halogenierten Derivat der Formel (III)

$$Hal - (CH_2)_n - R_1 \qquad (III)$$

zur Reaktion bringt, in der Hal ein Halogenatom darstellt, n die gleichen Bedeutungen wie in Formel (I) besitzt und $R_1$ die gleichen Bedeutungen wie vorstehend aufweist, daß man das Produkt isoliert und gegebenenfalls in ein Additionssalz mit einer Mineralsäure oder organischen Säure umwandelt,

B - zur Herstellung der Verbindungen der Formel (I), mit Ausnahme der Verbindungen, in denen $R_1$ einen 4-Aminophenyl-1-piperazinyl-Rest darstellt, ein Derivat der Formel (VI)

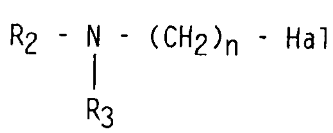 (VI)

in der n, $R_2$ und $R_3$ die gleichen Bedeutungen wie in Formel (I) besitzen und Hal ein Halogenatom darstellt, mit einem Derivat der Formel (IV)

$HR_1$ (IV)

zur Reaktion bringt, in der $R_1$ die gleichen Bedeutungen wie vorstehend aufweist, daß man das Produkt isoliert und gegebenenfalls in ein Additionssalz mit einer Mineralsäure oder organischen Säure umwandelt,

C - zur Herstellung der Verbindungen der Formel (I), in der $R_1$ einen 4-Aminophenyl-1-piperazinyl-Rest darstellt, die Verbindung der Formel (I), in der $R_1$ einen 4-Nitrophenyl-1-piperazinyl-Rest bedeutet, reduziert und man das Produkt isoliert und gegebenenfalls in ein Additionssalz mit einer Mineralsäure oder organischen Säure umwandelt.

2. Verfahren nach Anspruch 1 zur Herstellung der Verbindungen der Formel (I), in der $R_1$ einen 4-Phenyl-1,2,3,6-tetrahydro-1-pyridyl-, 4-Phenyl-piperidino-, 4-(4-Fluorbenzoyl)-piperidino- oder 4-Phenyl-1-piperazinyl-Rest darstellt, deren Phenylkern gegebenenfalls durch ein Halogenatom oder einen Hydroxyrest substituiert ist, $R_2$, $R_3$ und n die gleichen Bedeutungen wie in Anspruch 1 besitzen, sowie ihren Salzen mit Mineralsäuren oder organischen Säuren.

3. Verfahren nach Anspruch 1 zur Herstellung der Verbindungen der Formel (I) in der n gleich 3 ist.

4. Verfahren nach Anspruch 1 zur Herstellung der Verbindungen der Formel (I). in der $R_2$ und $R_3$ zusammen mit dem Stickstoffatom, an das sie gebunden sind, einen 5,6-Dihydro-1H,4H-1,2,5-thiadiazolo[4,3,2-ij]-chinolin-2,2-dioxid- oder 3-Phenyl-1,2-benzothiazol-1,1-dioxid-Ring bilden.

## Claims

### Claims for the following Contracting States: AT, BE, CH, DE, DK, FR, GB, IT, LI, LU, NL, SE

1. Compounds of formula:

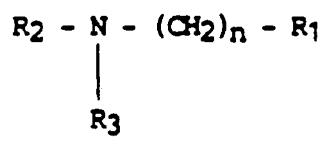

(I)

in which

- $R_1$ denotes
  - a 1,2,3,6-tetrahydro-1-pyridyl radical substituted in the 4- position by (a) a phenyl radical, (b) a phenyl radical substituted by a halogen atom or an alkyl, hydroxyl or alkoxy radical, (c) a 3-indolyl radical, (d) a 3-indolyl radical substituted on the nitrogen atom by an alkyl or alkylcarbonyl radical and/or in the 5- position by a chlorine or fluorine atom or (e) a 3-(5-hydroxyindolyl) radical,
  - a 1-piperazinyl radical substituted in the 4-position by (a) a phenyl radical, (b) a phenyl radical substituted by an alkoxy, alkyl, hydroxyl, nitro or amino radical or a halogen atom, (c) a 1,2-benzisothiazol-3-yl radical, (d) a 1,2-benzisoxazol-3-yl radical or (e) a 2-pyridyl radical,
  - a piperidino radical substituted in the 4-position by (a) a phenyl radical, (b) a phenyl radical substituted by a halogen atom or a hydroxyl, alkyl or alkoxy radical, (c) two phenyl radicals, (d) a bis(4-fluorophenyl)methylene radical, (e) a 4-fluoro- benzoyl radical, (f) a 2-oxo-1-benzimidazolinyl radical, (g) a 2-oxo-1-benzimidazolinyl radical substituted in the 3- position by an alkylcarbonyl or benzoyl radical, (h) a hydroxyl radical and a phenyl radical optionally substituted by an alkyl, alkoxy or hydroxyl radical or a halogen atom, (i) a 3-indolyl radical, (j) a 3-indolyl radical substituted on the nitrogen atom by an alkyl or alkylcarbonyl radical and/or in the 5- position by a chlorine or fluorine atom or (k) a 3-(5-hydroxyindolyl) radical,
- $R_2$ denotes a radical $SO_2R_4$ in which $R_4$ denotes an alkyl or phenyl radical,
- $R_3$ denotes a phenyl or naphthyl radical,
- or else $R_2$ and $R_3$ together with the nitrogen atom to which they are attached form a ring chosen from the formulae:

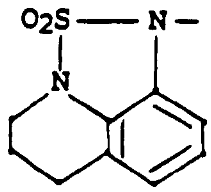

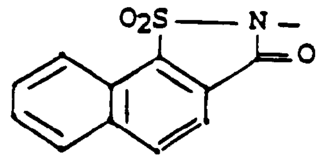

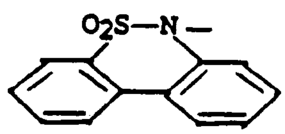

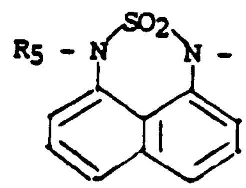

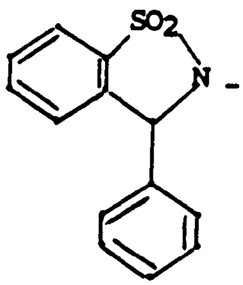

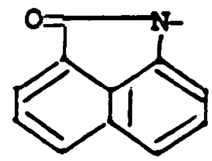

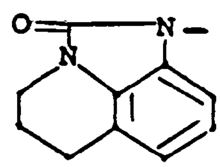

- $R_5$ denotes an alkyl radical or a $-(CH_2)_n-R_1$ chain,
- n is equal to 2, 3 or 4,

and their salts with inorganic or organic acids, it being understood that the alkyl and alkoxy radicals or the alkyl parts contain from 1 to 4 carbon atoms as a straight or branched chain.

2. Compounds of formula (I) according to Claim 1, in which $R_1$ denotes a 4-phenyl-1,2,3,6-tetrahydro-1-pyridyl, 4-phenylpiperidino, 4-(4-fluorobenzoyl)piperidino or 4-phenyl-1-piperazinyl radical whose phenyl nucleus is optionally substituted by a halogen atom or a hydroxyl radical, $R_2$, $R_3$ and n have the same meanings as in Claim 1, and their salts with inorganic or organic acids.

3. Compounds of formula (I) according to Claims 1 or 2, in which n is equal to 3.

4. Compounds of formula (I) according to one of Claims 1 to 3, in which $R_2$ and $R_3$ together with the nitrogen atom to which they are attached form a 5,6-dihydro(1H, 4H)-1,2,5-thiadiazolo[4,3,2-ij]quinoline 2,2-dioxide or 3-phenyl-1,2-benzothiazole 1,1-dioxide ring.

5. Process for the preparation of the compounds of formula (I) according to Claim 1, with the exception of those in which $R_1$ denotes a 4-aminophenyl-1-piperazinyl radical, characterized in that a derivative of formula:

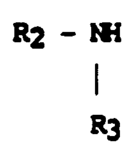

(II)

in which $R_2$ and $R_3$ have the same meanings as in Claim 1, is reacted with a halogenated derivative of formula:

$$Hal - (CH_2)_n - R_1 \qquad (III)$$

in which Hal denotes a halogen atom, n has the same meanings as in Claim 1 and $R_1$ has the same meanings as above, and the product is isolated and optionally converted into an addition salt with an inorganic

or organic acid.

6. Process for the preparation of the compounds of formula (I) according to Claim 1, with the exception of those in which $R_1$ denotes a 4-aminophenyl-1-piperazinyl radical, characterized in that a derivative of formula:

$$R_2 - N(R_3) - (CH_2)_n - Hal \quad (VI)$$

is reacted with a derivative of formula:

$$HR_1 \quad (IV)$$

in which $R_1$ has the same meanings as above, $R_2$, $R_3$ and n have the same meanings as in Claim 1 and Hal denotes a halogen atom, and the product is isolated and optionally converted into an addition salt with an inorganic or organic acid.

7. Process for the preparation of the compounds of formula (I) according to Claim 1, in which $R_1$ denotes a 4-aminophenyl-1-piperazinyl radical, characterized in that the corresponding compound of formula (I) in which $R_1$ denotes a 4-nitrophenyl-1-piperazinyl radical is reduced and the product is isolated and optionally converted into an addition salt with an inorganic or organic acid.

8. Medications which contain as active substance at least one compound of formula (I) according to Claim 1.

9. Medications according to Claim 8 for the treatment of illnesses where serotonin is involved.

**Claims for the following Contracting States : ES, GR**

1. Process for the preparation of compounds of formula:

$$R_2 - N(R_3) - (CH_2)_n - R_1 \quad (I)$$

in which

- $R_1$ denotes
  - a 1,2,3,6-tetrahydro-1-pyridyl radical substituted in the 4- position by (a) a phenyl radical, (b) a phenyl radical substituted by a halogen atom or an alkyl, hydroxyl or alkoxy radical, (c) a 3-indolyl radical, (d) a 3-indolyl radical substituted on the nitrogen atom by an alkyl or alkylcarbonyl radical and/or in the 5- position by a chlorine or fluorine atom or (e) a 3-(5-hydroxyindolyl) radical,
  - a 1-piperazinyl radical substituted in the 4-position by (a) a phenyl radical, (b) a phenyl radical substituted by an alkoxy, alkyl, hydroxyl, nitro or amino radical or a halogen atom, (c) a 1,2-benzisothiazol-3-yl radical, (d) a 1,2-benzisoxazol-3-yl radical or (e) a 2-pyridyl radical,
  - a piperidino radical substituted in the 4-position by (a) a phenyl radical, (b) a phenyl radical substituted by a halogen atom or a hydroxyl, alkyl or alkoxy radical, (c) two phenyl radicals, (d) a bis(4-fluorophenyl)methylene radical, (e) a 4-fluorobenzoyl radical, (f) a 2-oxo-1-benzimidazolinyl radical, (g) a 2-oxo-1-benzimidazolinyl radical substituted in the 3-position by an alkylcarbonyl or benzoyl radical, (h) a hydroxyl radical and a phenyl radical optionally substituted by an alkyl, alkoxy or hydroxyl radical or a halogen atom, (i) a 3-indolyl radical, (j) a 3-indolyl radical substituted on the nitrogen atom by an alkyl or alkylcarbonyl radical and/or in the 5- position by a chlorine or fluorine atom or (k) a 3-(5-hydroxyindolyl) radical,
- $R_2$ denotes a radical $SO_2R_4$ in which $R_4$ denotes an alkyl or phenyl radical,
- $R_3$ denotes a phenyl or naphthyl radical,
- or else $R_2$ and $R_3$ together with the nitrogen atom to which they are attached form a ring chosen from the formulae:

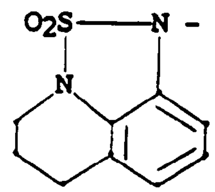

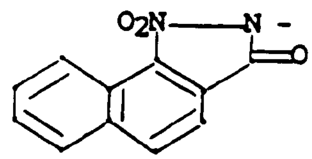

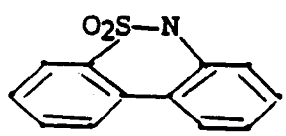

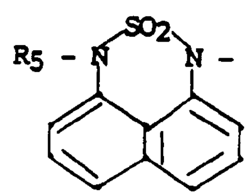

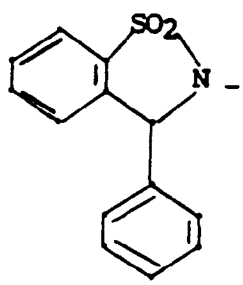

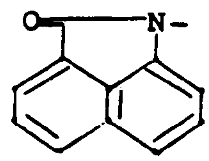

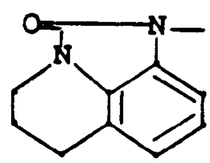

- $R_5$ denotes an alkyl radical or a $-(CH_2)_n-R_1$ chain,
- n is equal to 2, 3 or 4 and their salts with inorganic or organic acids, characterized in that

A- for the preparation of the compounds of formula (I) with the exception of the compounds in which $R_1$ denotes a 4-aminophenyl-1-piperazinyl radical, a derivative of formula:

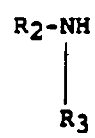

(II)

in which $R_2$ and $R_3$ have the same meanings as above, is reacted with a halogenated derivative of formula:

$$Hal-(CH_2)_n-R_1 \qquad (III)$$

in which Hal denotes a halogen atom, n has the same meanings as in formula (I) and $R_1$ has the same meanings as above, and the product is isolated and is optionally converted into an addition salt with an inorganic or organic acid,

B - for the preparation of the compounds of formula (I) with the exception of those in which $R_1$ denotes a 4-aminophenyl-1-piperazinyl radical, a derivative of formula

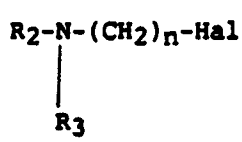

(VI)

in which n, $R_2$ and $R_3$ have the same meanings as in formula (I) and Hal denotes a halogen atom, is reacted with a derivative of formula:

$$HR_1 \qquad (IV)$$

in which $R_1$ has the same meanings as above and the product is isolated and optionally converted into an addition salt with an inorganic or organic acid,

C - for the preparation of the compounds of formula (I) in which $R_1$ denotes a 4-aminophenyl-1-piperazinyl radical, the corresponding compound of formula (I) in which $R_1$ denotes a 4-nitrophenyl-1-piperazinyl radical is reduced and the product is isolated and optionally converted into an addition salt with an inorganic or organic acid.

2. Process according to Claim 1 for the preparation of compounds of formula (I) in which $R_1$ denotes a 4-phenyl-1,2,3,6-tetrahydro-1-pyridyl, 4-phenylpiperidino, 4-(4-fluorobenzoyl)piperidino or 4-phenyl-1-piperazinyl radical whose phenyl nucleus is optionally substituted by a halogen atom or a hydroxyl radical, $R_2$, $R_3$ and n have the same meanings as in Claim 1, and their salts with inorganic or organic acide.

3. Process according to Claim 1 for the preparation of compounds of formula (I) in which n is equal to 3.

4. Process according to Claim 1 for the preparation of compounds of formula (I) in which $R_2$ and $R_3$ together with the nitrogen atom to which they are attached form a 5,6-dihydro[1H,4H]-1,2,5-thiadiazolo[4,3,2-ij]quinoline 2,2-dioxide or 3-phenyl-1,2-benzothiazole 1,1-dioxide ring.